# EUROPEAN PATENT SPECIFICATION

(11) **EP 0 534 548 B1**
(45) Date of publication and mention of the grant of the patent: **21.01.1998**
(21) Application number: 92202882.4
(22) Date of filing: 21.09.1992
(51) Int. Cl.: A61B 6/14, A61B 6/00

(54) **Cephalostat**
Kephalostat
Céphalostat

(30) Priority: 27.09.1991 EP 91202533
(43) Date of publication of application: 31.03.1993
(73) Proprietor: Dentsply Research & Development Corp., Milford Delaware 19963 (US)
(72) Inventor: Molteni, Roberto, NL-5656 AA Eindhoven (NL); Lambrecht, Jörg Thomas, NL-5656 AA Eindhoven (NL)
(74) Representative: Gordon, Richard John Albert

(56) References cited:
- DE-U- 8 625 806
- US-A- 3 539 805
- US-A- 4 088 893
- US-A- 4 759 361

## Description

This invention relates to a cephalostat for stable, accurate and reproducible positioning of a patient's head for dental X-ray examination.

Such a device is known from EP-A-0 193 650 and US-A-3 539 805 and incudes means for Tempora-Mandibular Joint (TMJ) positioning with reference to associated rotational panoramic radiographic apparatus, the means extending upwardly from a mechanism thereby permitting rotatable movement and operation of the apparatus unfettered by the cephalostat, and a naison support for restraining the patient's head from turning in a mid sagittal plane.

However, a problem associated with such devices is that they are not sufficiently adaptable either because there is no means for restraining a patient's head movements or because the patient's head is so firmly positioned that it is not possible to make adjustments for carrying out special imaging methods.

The invention is characterised in that the naison support is attached to the cephalostat such that it is displaceable along a vertical axis and is pivotable on that axis to bring a patient's head into a fixed examination position.

Therefore, a cephalostat in accordance with the present invention, whether constructed as a separate device or incorporated in dental X-ray examination apparatus, provides for enhanced adaptability compared with cephalostats known hitherto and, in particular, provides for the possibility of examinations with mandibular movements and thus opening the possibility for congruent imaging of closed and open mouth TMJ's.

A stable accurate and reproducible positioning with respect to a mechanical part of the apparatus which is inherently a geometrical reference between a patient's head anatomy, and via the apparatus construction between the beam path of an imaging X-ray beam such as for example the scanning geometrical pattern of a laminar X-ray beam to be generated in the apparatus.

In a preferred embodiment a cephalostat is provided with ear plugs for Tempora-Mandibular-Joint (TMJ) positioning and a frontal support immobilizing the upper jaw without mandible immobilizing. With such fixing means an accurate positioning for TMJ examinations has been realised together with the possibity to make congruent examination under different positions of the mandible with respect to the upper jaw. Preferably the mutual distance of the ear plugs is adjustable for fixing and adapting to heads of different width. During this adjustment a common midpoint of the ear plugs, with are preferably fixed to the patient's ear meati, remains stable with respect to the cephalostat and thus with respect to the X-ray apparatus and more specifically to the X-ray beam path.

In a further embodiment frontal positioning is performed with the aid of a nose support. Herewith the patient upper head part become fixed with respect to the common midpoint of the ear plug in an accurate predefined position because that midpoint together with a fixed nose point exactly defines the position of relevant parts to be examined. The nose support is preferably adjustable with respect to the ear midpoint as well for mounting as for adjusting to relevant head sizes.

In a preferred embodiment the cephalostat is constructed as a separate unit being mechanically mountable to a dental X-ray apparatus such as a dento-maxillo-facial X-ray apparatus in a well defined fixed and reproducible manner. Preferably the cephalostat and the X-ray apparatus are provided with reference planes or points assuring a right positioning.

In a further embodiment the cephalostat forms part of a dental X-ray apparatus being preferably mounted therein in a fixed and exact position relative to a beam path of an imaging X-ray beam to be generated in the apparatus.

With a cephalostat according to the invention positioning of a patient's head can be executed quick and confortable for the patient irrespective from the head size. Starting with the ear plugs and the nasion support in an all-open position they can simply be closed until they rest in proper position. The patient's head only makes two movements, first displacement enabling the ear plugs to enter into the ears and subsequently a tilting until the frontal support rests at the nose.

The positioning is very accurate and well defined in that the ear plugs in fact define the position of the jaws into the apparatus relative to the acoustic meatus which are very near to the anatomic detail to be examined such as for TMJ imaging. Different angulation of the head relative to the horizontal plane, for different diagnostic needs, can be achieved either using nasion supports of different length, thus at different height with respect to the ear plugs position, or resting the support at a different anatomic point e.g. the subspinal point.

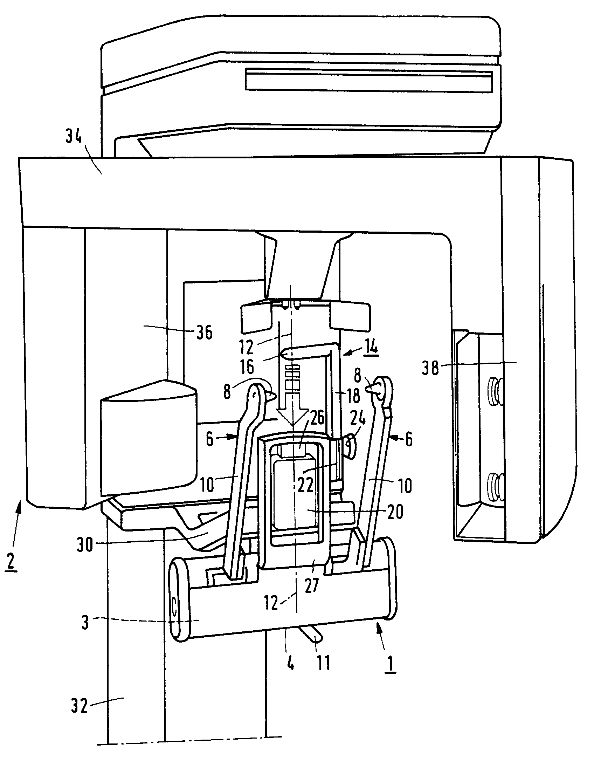

Embodiments of the invention will be described by way of example in more detail in the following with reference to the accompanying drawing.

In the sole figure of the drawing a cephalostat 1 according to the invention is given in a position relative to a dental X-ray examination apparatus 2. The cephalostat comprises a mechanical moving mechanism 3 enclosed in a box 4 such that no interaction between a patient and moving parts can occur. The cephalostat further comprises two ear plugs 6 with contacting buttons 8 and armes 10. Lower parts of the arms are connected with the moving mechanism 3 such that their mutual distance can be varied. By this spacing variation both ear plugs remain on a mutually common distance from a midplane 12 or as can also be said the ear plugs move symmetrically relative to said midplane 12. The distance variation mechanism can be locked and unlocked with a lever 11. The cephalostat as given shows further a frontal support in the shape of a nasion support 14. The nasion support 14 is provided with a nose contacting member 16 a supporting arm 18 being connected to a central support 20 with a pivot connection 22 to be locked and unlocked with a knob 24 such that the nose contact member can be angulated in order to create room to bring a patient's head in an examination position. The central support 20 is provided with a plug 26 and a frame 27 for fixing the cephalostat to the X-ray apparatus in an accurate position preferably interchangeable in an easy manner. The nose contacting member 16 can also be moved up and down with respect to the central support in order to adapt to the nose position of a given patient to be examined. The cephalostat 1 is fixed to a dental X-ray apparatus 2, preferably for example an orthophantographic apparatus in a well defined position with respect to a beam path for an X-ray beam to be generated therein. This can be realised in a simple manner in that clamping means 30 to which the central support 20 can be fixed are the same or alternative for clamping a chin rest or bite block. The clamping means 30 are coupled to a column 32 of the apparatus to which in a known manner a support 34 for an X-ray tube casing 36 and X-ray detecting means such as a film holder 38. For dental apparatus to be used explicitaly for rotational panoramic radiography the cephalostat is preferably part of the apparatus avoiding any misorientation in mutual positioning but in general the cephalostat is constructed as an individual device to be clamped interchangeable to any relevant dental X-ray apparatus.

## Claims

1. A cephalostat (1) for stable, accurate and reproducible positioning of a patient's head for dental X-ray examination including means (6) for Tempora-Mandibular Joint (TMJ) positioning with reference to associated rotational panoramic apparatus (2), the said means (6) extending upwardly from a mechanism (3) thereby permitting rotatable movement and operation of the apparatus (2) unfettered by the cephalostat (1), and a naison support (14) for restraining the patient's head from turning in a mid sagittal plane characterised in that the naison support is attached to the cephalostat (1) such that it is displaceable along a vertical axis and is pivotable on that axis to bring the patient's head into a fixed examination position.

2. A cephalostat (1) as claimed in Claim 1 characterised in that the naison support (14) is attached to the cephalostat (1) by means of a pivot connection (22).

3. A cephalostat (1) as claimed in Claim 2 characterised in that there is provided means (24) for locking the naison support in the examination position.

## Patentansprüche

1. Kephalostat (1) zur stabilen, genauen und reproduzierbaren Positionierung des Kopfes eines Patienten zur dentalen Röntgenstrahluntersuchung, welche eine Einrichtung (6) aufweist zur Positionierung der Tempora-Mandibularen- bzw. Unterkieferverbindung (TMJ) bezüglich einer damit verbundenen rotatorischen Panoramavorrichtung (2), wobei die Einrichtung (6) sich von einem Mechanismus (3) nach oben erstreckt, und dadurch eine Drehbewegung und einen Drehbetrieb der Vorrichtung (2) ermöglicht, und zwar unabhängig von dem Kephalostat (1) und eine Nasenstützeinrichtung (14) zum Zurückhalten des Kopfes des Patienten von einem Drehen in eine mittlere Körperebene,
**dadurch gekennzeichnet,** daß
die Nasenstützeinrichtung derart an das Kephalostat (1) angebracht ist, daß sie entlang einer vertikalen Achse verschiebbar ist, und daß sie um diese Achse gelenkig ist, um den Kopf des Patienten in eine feste Untersuchungsposition zu bringen.

2. Kephalostat (1) nach Anspruch 1,
**dadurch gekennzeichnet,** daß
die Nasenstützeinrichtung an das Kephalostat (1) mittels einer Gelenkverbindung (22) angebracht ist.

3. Kephalostat (1) nach Anspruch 2,
**dadurch gekennzeichnet,** daß
eine Einrichtung (24) zum Blockieren der Nasenstützeinrichtung in der Untersuchungsposition vorgesehen ist.

## Revendications

1. Céphalostat (1) pour un positionnement stable, efficace et reproductible de la tête d'un patient pour un examen dentaire par rayons X, comportant des moyens (6) de positionnement du joint temporal-mandibulaire (JTM) en référence à un appareil panoramique rotatif associé (2), lesdits moyens (6) s'étendent vers le haut depuis un mécanisme (3), permettant ainsi un mouvement rotatif et une utilisation de l'appareil (2) non entravé par le céphalostat (1), et un support nasal (14) pour empêcher la tête du patient de se tourner dans un plan sagital médian, caractérisé en ce que le support nasal est fixé au céphalostat (1) de telle façon qu'il puisse être déplacé le long d'un axe vertical et qu'il puisse être pivoté sur cet axe pour amener la tête du patient dans une position d'examen fixe.

2. Céphalostat (1) selon la revendication 1, caractérisé en ce que le support nasal (14) est fixé au céphalostat (1) au moyen d'une connexion à pivot (22).

3. Céphalostat (1) selon la revendication 2, caractérisé en ce qu'il comporte des moyens (24) pour verrouiller le support nasal dans la position d'examen.
